# EUROPÄISCHE PATENTSCHRIFT

(11) **EP 0 659 173 B1**
(45) Veröffentlichungstag und Bekanntmachung des Hinweises auf die Patenterteilung: **03.12.1997**
(21) Anmeldenummer: 93919214.2
(22) Anmeldetag: 30.08.1993
(51) Int. Cl.: C07C 209/86, C07C 209/36

(54) **VERFAHREN ZUR HERSTELLUNG VON RÜCKSTANDSFREIEM 2,4/2,6-DIAMINOTOLUOLGEMISCH**
METHOD OF MANUFACTURING A RESIDUE-FREE 2,4/2,6-DIAMINOTOLUENE MIXTURE
PROCEDE DE PREPARATION D'UN MELANGE DE 2,4/2,6-DIAMINOTOLUENES SANS RESIDU

(30) Priorität: 09.09.1992 DE 4230098
(43) Veröffentlichungstag der Anmeldung: 28.06.1995
(73) Patentinhaber: BAYER AG, 51368 Leverkusen (DE)
(72) Erfinder: ZARNACK, Uwe, Jens, D-25541 Brunsbüttel (DE); DIESSELKÄMPER, Bernd, D-25709 Marne (DE); RINDFLEISCH, Hans-Nicolaus, D-25541 Brunsbüttel (DE)
(86) Internationale Anmeldenummer: EP9302335
(87) Internationale Veröffentlichungsnummer: WO9406752

(56) Entgegenhaltungen:
- KIRK-OTHMER 'Encyclopedia of Chemical Technology' 1978 , J.WILEY & SONS , NEW YORK ETC. siehe Seite 324 - Seite 327

## Beschreibung

Die vorliegende Erfindung betrifft ein Verfahren zur kontinuierlichen Abtrennung von Rückstand aus rohen m-Diaminotoluolgemischen (m-TDA; 2,4/2,6-Diaminotoluolgemisch) zur Herstellung von rückstandsfreien 2,4/2,6-Diaminotoluolgemischen (m-TDA).

Bei der großtechnischen Produktion von m-TDA durch Dinitrierung von Toluol und anschließender Hydrierung zu den Toluylendiaminen entstehen naturgemäß höhersiedende Nebenprodukte, u.a. Diphenylmethane, Diphenylamine, Acridine und Phenazine.

Das aus Toluol durch Dinitrierung und anschließende Hydrierung produzierte m-Toluylendiamingemisch enthält je nach Herstellungsverfahren zwischen 0,3 % und 2 % höhermolekulare Nebenkomponenten, im folgenden als TDA-Rückstand bezeichnet. Nach der destillativen Abtrennung der Leichtsieder Lösemittel, Wasser und den ortho-Isomeren (2,3- und 3,4-Diaminotololen) sind drei Verfahren zur Weiterverarbeitung des rückstandshaltigen m-TDA-Gemisches bekannt.
1. Das rückstandshaltige m-TDA-Gemisch wird direkt zu m-Toluylendiisocyanat (TDI) phosgeniert. Die dabei mitphosgenierten höhermolekularen Verbindungen werden im Aufarbeitungsprozeß als TDI-Rückstand ausgeschleust.
   - Vorteil:: - Einsparung eines Verfahrensschrittes
   - Nachteile:: - Das Verfahren ist nur auf Flüssigphasenphosgenierung anwendbar
   - Rückstand im m-TDA führt im Folgeprozeß zur verstärkten Bildung höhermolekularer Komponenten auf Kosten der Ausbeute
   - Störungen in der Rückstandsaustragung des TDI-Prozesses
2. Das rückstandshaltige m-TDA-Gemsich wird in mit 30 bar Dampf beheizten Destillationsblasen über eine Kolonne ausdestilliert. Damit der Sumpf unter diesen Bedingungen (230°C) fließfähig ist, verbleibt ein Anteil m-TDA > 5 % im Sumpf.
   - Vorteil:: - Das rückstandsfreie m-TDA ist einsetzbar in der Gasphasenphosgenierung und als Verkaufsprodukt.
   - Nachteile:: - Geringere Ausbeute als beim Verfahren unter Punkt 1.
   - Entsorgung des TDA-Rückstandsgemisches (hochviskos) zur Verbrennung unter Verlust von m-TDA
3. Kontinuierliche Rückstandsabtrennung durch Resteindampfung des rückstandshaltigen m-TDA im Dünnschichtverdampfer und Ausschleusung des Rückstandes mit einem m-TDA Anteil > 10 % als "Weichmacher".
   - Vorteil:: - Das rückstandsfreie m-TDA ist einsetzbar in der Gasphasenphosgenierung und als Verkaufsprodukt.
   - Nachteile:: - Geringere Ausbeute als beim Verfahren unter Punkt 1.
   - Entsorgung des TDA-Rückstandsgemisches (hochviskos) zur Verbrennung unter Verlust von m-TDA.

Bei der destillativen Abtrennung des Rückstandes gemäß den Verfahren unter Punkt 2. und 3. fällt dieser als hochviskose Masse an, die - abhängig vom Restgehalt an m-TDA - bei Temperaturen unter 150°C glasartig erstarrt. Das im Rückstand verbleibende m-TDA geht zulasten der Ausbeute des Prozesses.

Darüber hinaus ist aus Kirk-Othmer, 3rd Ed. Band 2 (1978), Seiten 326 - 327 ein kontinuierliches Verfahren zur Rückstandsaufarbeitung (Sumpfausschleusung) beschrieben, das auf Patentveröffentlichungen von Allied Chemical basiert. Dabei wird in der letzten Kolonne m-TDA, das über Kopf abdestilliert wird, vom Rückstand, der als Sumpf ausgeschleust wird, abgetrennt. Die Vorteile dieses Aufarbeitungsverfahrens entsprechen dem unter Punkt 2 genannten Aufarbeitungsverfahren; ebenso wie die Nachteile.

Aufgabe war es daher, ein Verfahren zur Verfügung zu stellen, das es gestattet, rückstandsfreies m-TDA herzustellen und vollständig aus dem rohen m-TDA bzw. dem Rückstand abzutrennen, ohne daß dabei die o.g. Nachteile auftreten.

Diese Aufgabe konnte mit dem erfindungsgemäßen Verfahren gelöst werden.

Gegenstand der Erfindung ist ein Verfahren zur Herstellung von rückstandsfreiem m-Toluylendiamin durch Nitrierung von Toluol, Hydrierung der Nitroverbindungen, destillative Abtrennung des Wassers und des Lösungsmittels, sowie destillative Entfernung der ortho-Isomeren aus dem Toluylendiamingemisch und anschließende Destillation des verbleibenden Restes, welches dadurch gekennzeichnet ist, daß die Destillation so durchgeführt wird, daß so viel 2,4- und 2,6-Toluylendiamine (m-Toluylendiamin, m-TDA) aus dem verbleibenden Rest abdestilliert werden, daß ein Sumpf mit einer Rückstandskonzentration von 30 bis 70 Gew.-% verbleibt, dieser Sumpf mit einem gegenüber Toluylendiaminen inerten Hilfsmittel mit einem Siedepunkt von > 290°C in einer Menge abgemischt wird, daß sich eine Rückstandskonzentration von 28 bis 50 Gew.-% einstellt, dieser mit Hilfsmittel versetzte Sumpf solange einer weiteren ein-oder mehrstufigen Destillation unterzogen wird, bis eine Restkonzentration an 2,4- und 2,6-Toluylendiamin von 1 bis 5 Gew.-% im Sumpf erreicht ist, und der verbleibende restliche, auch bei niedrigen Temperaturen noch flüssige Sumpf ausgeschleust wird.

Der Vorteil des erfindungsgemäßen Verfahrens ist, daß ohne großen technischen Aufwand fast das gesamte m-TDA rückstandsfrei erhalten werden kann und daS außerdem der verbleibende Rückstand selbst bei niedrigen Temperaturen (ca. 100°C) ohne Probleme ausgeschleust werden kann.

Bevorzugt werden als Hilfsmittel eine oder mehrere Verbindungen aus der Gruppe
- Destillationsrückstande aus der Ethylenglykolherstellung (EDR)
- Destillationsrückstande aus der Propylenglykolherstellung (PDR)
- Hochsiedende Polyether auf Basis Ethylenoxid
- Hochsiedende Polyether auf Basis Propylenoxid
- Hochsiedende Polyether auf Basis Mischpolymerisate aus Ethylen- und Propylenoxid
- Hochsiedende Polyether auf Basis Polytetrahydrofurane
- Hochsiedende Polyether auf Basis Mischpolymerisate aromatischer Hydroxylverbindungen
- Hochsiedende Polyether auf Basis cycloaliphatischer Oxoverbindungen
- Zucker, z.B. Melasse
- Hochsiedende Alkylaromaten
eingesetzt.

Vorzugsweise wird das erfindungsgemäße Verfahren kontinuierlich durchgeführt. Das rohe m-TDA (Toluylendiamingemisch nach Abtrennung der Leichtsieder (Wasser, Lösungsmittel) und der ortho-Isomeren) enthält normalerweise 0,3 bis 2 Gew.-% Rückstand. In einem ersten Schritt wird soviel m-TDA (2,4/2,6-Toluylendiamin) abdestilliert, daß anschließend die Konzentration des Rückstands 30 bis 70 Gew.-% betragt. Dieses aufkonzentrierte rohe m-TDA wird mit einem Hilfsstoff abgemischt und einer zweiten Destillation unterzogen. Vorzugsweise wird dabei bei Temperaturen zwischen 160 und 260°C, besonders bevorzugt zwischen 190 und 220°C, und bei Drucken zwischen 2 und 100 mbar, besonders bevorzugt zwischen 5 und 40 mbar, gearbeitet. Nach der Destillation beträgt die Konzentration an m-TDA im Sumpf bevorzugt nur noch 1 bis 5 Gew.-%. Der Sumpf wird ausgeschleust und hat vorzugsweise eine Viskosität bei 100°C von ≤ 150 mPas.

Die Erfindung soll anhand des nachfolgenden Beispiels näher erläutert werden.

### Beispiel

152,733 kg rohes m-TDA mit einem Rückstandsgehalt von 1,5 % werden kontinuierlich in eine Destillationskolonne die bei 206°C und 5 mbar betrieben wird, eingespeist. Als Destillat werden 148,649 kg m-TDA erhalten (Ausbeute bezogen auf m-TDA: 98,81 %). Das aus der Destillationskolonne ablaufende Rückstandskonzentrat (4,0842 kg; Rückstandsgehalt: 56,1 %) wird mit 5,325 kg Ethylenglykoldestillationsrückstand abgemischt und kontinuierlich in eine zweite Kolonne, die bei 206°C und 5 mbar betrieben wird, eingespeist. Als Destillat werden 1,724 kg m-TDA erhalten (Ausbeute bezogen auf m-TDA: 1,15 %). Die Gesamtausbeute an m-TDA beträgt 99,95 %.

Der aus der Kolonne ablaufende Rückstand enthält noch 0,05 % m-TDA (bezogen auf die Gesamtmenge m-TDA) und hat bei 80°C eine Viskosität von 50 mPas.

## Patentansprüche

1. Verfahren zur Herstellung von rückstandsfreiem m-Toluylendiamin durch Nitrierung von Toluol, Hydrierung der Nitroverbindungen, destillative Abtrennung des Wassers und des Lösungsmittels sowie destillative Entfernung der ortho-Isomeren aus dem rückstandhaltigen Toluylendiamingemisch und anschließende Destillation des verbleibenden Restes, dadurch gekennzeichnet, daß die Destillation so durchgeführt wird, daß so viel 2,4-und 2,6-Toluylendiamine (m-Toluylendiamin, m-TDA) aus dem verbleibenden Rest abdestilliert werden, daß ein Sumpf mit einer Rückstandskonzentration von 30 bis 70 Gew.-% verbleibt, dieser Sumpf mit einem gegenüber Toluylendiaminen inerten Hilfsmittel, mit einem Siedepunkt von > 290°C in einer Menge abgemischt wird, daß sich eine Rückstandskonzentration von 28 bis 50 Gew.-% einstellt, dieser mit Hilfsmittel versehene Sumpf solange einer weiteren ein- oder mehrstufigen Destillation unterzogen wird, bis eine Restkonzentration an 2,4- und 2,6-Toluylendiamin von 1 bis 5 Gew.-% in Sumpf erreicht ist, und der verbleibende restliche, auch bei niedrigen Temperaturen noch flüssige Sumpf ausgeschleust wird.

2. Verfahren gemäß Anspruch 1, dadurch gekennzeichnet, daß als Hilfsmittel eine oder mehrere Verbindungen aus der Gruppe
- Destillationsrückstände aus der Ethylenglykolherstellung (EDR)
- Destillationsrückstände aus der Propylenglykolherstellung (PDR)
- Hochsiedende Polyether auf Basis Ethylenoxid
- Hochsiedende Polyether auf Basis Propylenoxid
- Hochsiedende Polyether auf Basis Mischpolymerisate aus Ethylen- und Propylenoxid
- Hochsiedende Polyether auf Basis Polytetrahydrofurane
- Hochsiedende Polyether auf Basis Mischpolymerisate aromatischer Hydroxylverbindungen
- Hochsiedende Polyether auf Basis cycloaliphatischer Oxoverbindungen
- Zucker, z.B. Melasse
- Hochsiedende Alkylaromaten
eingesetzt werden.

3. Verfahren gemäß Anspruch 1, dadurch gekennzeichnet, daß die Destillation des verbleibenden Restes ein- oder mehrstufig erfolgt.

4. Verfahren gemäß Anspruch 1, dadurch gekennzeichnet, daß die Destillation bei Temperaturen von 160°C bis 260°C, bevorzugt bei Temperaturen von 190°C bis 220°C, und bei Drucken von 2 bis 100 mbar, bevorzugt 5 bis 40 mbar, durchgeführt werden.

## Claims

1. Process for the preparation of residue-free m-tolylenediamine by nitrating toluene, hydrogenating the nitro compounds, separating the water and solvent by distillation and removing the ortho-isomers from the residue-containing tolylenediamine mixture by distillation, followed by distillation of the remainder, characterised in that the distillation is performed such that a quantity of 2,4- and 2,6-tolylenediamine (m-tolylenediamine, m-TDA) is distilled from the remainder such that a bottom having a residue concentration of from 30 to 70 wt-% remains, the latter bottom is mixed with an auxiliary substance which is inert to tolylenediamines and has a boiling point of > 290°C in a quantity such as to adjust a residue concentration of from 28 to 50 wt-%, the latter bottom which is provided with the auxiliary substance undergoes a further single- or multi-stage distillation until a residual 2,4- and 2,6-tolylenediamine concentration of from 1 to 5 wt-% is obtained in the bottom, and the remaining residual bottom, which is still liquid even at low temperatures, is transferred out.

2. Process according to Claim 1, characterised in that one or more compounds from the following group are utilised as the auxiliary substance:
- distillation residues from ethylene glycol preparation (EDR)
- distillation residues from propylene glycol preparation (PDR)
- high-boiling polyethers based on ethylene oxide
- high-boiling polyethers based on propylene oxide
- high-boiling polyethers based on mixed polymers of ethylene oxide and propylene oxide
- high-boiling polyethers based on polytetrahydrofurans
- high-boiling polyethers based on mixed polymers of aromatic hydroxyl compounds
- high-boiling polyethers based on cycloaliphatic oxo compounds
- sugar, for example molasses
- high-boiling alkyl aromatics.

3. Process according to Claim 1, characterised in that the distillation of the remainder takes place in single- or multi-stage manner.

4. Process according to Claim 1, characterised in that the distillation is performed at temperatures of from 160°C to 260°C, preferably at temperatures of from 190°C to 220°C, and at pressures of from 2 to 100 mbar, preferably from 5 to 40 mbar.

## Revendications

1. Procédé pour préparer une m-toluylènediamine exempte de résidu par nitration du toluène, hydrogénation des dérivés nitrés, séparation par distillation de l'eau et du solvant et élimination par distillation des isomères ortho à partir du mélange de toluylènediamines contenant le résidu puis distillation de la masse restante, caractérisé en ce que l'on distille de la masse restante la 2,4- et la 2,6-toluylènediamines (m-toluylènediamine, m-TDA) en quantité telle qu'il subsiste un culot à une concentration en résidu de 30 à 70 % en poids, on mélange ce culot avec un produit auxiliaire inerte à l'égard des toluylènediamines et ayant un point d'ébullition supérieur à 290°C en quantité suffisante pour régler à une concentration en résidu de 28 à 50 % en poids, on soumet ce culot additionné du produit auxiliaire à une autre distillation à un ou plusieurs étages jusqu'à une concentration résiduelle de 1 à 5 % en poids de 2,4- et de 2,6-toluylènediamines dans le culot et on évacue le culot restant, toujours liquide même à basse température.

2. Procédé selon la revendication 1, caractérisé en ce que le produit auxiliaire consiste en un ou plusieurs composés du groupe suivant.
- résidus de distillation de la préparation de l'éthylèneglycol (EDR)
- résidus de distillation de la préparation du propylèneglycol (PDR)
- polyéthers à haut point d'ébullition à base d'oxyde d'éthylène
- polyéthers à haut point d'ébullition à base d'oxyde de propylène
- polyéthers à haut point d'ébullition à base de copolymères de l'oxyde d'éthylène et de l'oxyde de propylène
- polyéthers à haut point d'ébullition à base de polytétrahydrofuranes
- polyéthers à haut point d'ébullition à base de copolymères de dérivés aromatiques hydroxylés
- polyéthers à haut point d'ébullition à base de dérivés cycloaliphatiques en oxo
- sucres, par exemple mélasses
- composés alkylaromatiques à haut point d'ébullition.

3. Procédé selon la revendication 1, caractérisé en ce que la distillation de la masse restante est réalisée en un ou plusieurs stades.

4. Procédé selon la revendication 1, caractérisé en ce que la distillation est réalisée à des températures de 160 à 260°C, de préférence de 190 à 220°C sous des pressions de 2 à 100 mbar, de préférence de 5 à 40 mbar.
